(19) Europäisches Patentamt
European Patent Office
Office européen des brevets

(11) EP 1 961 415 A1

(12) EUROPEAN PATENT APPLICATION
published in accordance with Art. 158(3) EPC

(43) Date of publication:
27.08.2008 Bulletin 2008/35

(21) Application number: 06834630.3

(22) Date of filing: 13.12.2006

(51) Int Cl.:
*A61K 9/70* *(2006.01)* *A61K 47/10* *(2006.01)*
*A61K 47/32* *(2006.01)*

(86) International application number:
PCT/JP2006/324875

(87) International publication number:
WO 2007/069662 (21.06.2007 Gazette 2007/25)

(84) Designated Contracting States:
AT BE BG CH CY CZ DE DK EE ES FI FR GB GR HU IE IS IT LI LT LU LV MC NL PL PT RO SE SI SK TR

(30) Priority: 13.12.2005 JP 2005358469
06.12.2006 JP 2006328952

(71) Applicant: NITTO DENKO CORPORATION
Ibaraki-shi,
Osaka 567-8680 (JP)

(72) Inventors:
- IWAO, Yoshihiro
Ibaraki-shi, Osaka 567- 8680 (JP)
- OOKUBO, Katsuyuki
Ibaraki-shi, Osaka 567- 8680 (JP)
- OKADA, Katsuhiro
Ibaraki-shi, Osaka 567- 8680 (JP)

(74) Representative: Grünecker, Kinkeldey, Stockmair & Schwanhäusser
Anwaltssozietät
Leopoldstrasse 4
80802 München (DE)

# (54) ADHESIVE PREPARATION

(57) In the adhesive pharmaceutical preparation of the invention, a pressure-sensitive adhesive layer is laminated on one side of the backing. The pressure-sensitive adhesive layer contains a branched monoalcohol having from 12 to 28 carbon atoms, a drug which is liquid at room temperature or around room temperature (in which, free base of bisoprolol is excluded) and a polyisobutylene pressure-sensitive adhesive. Accordingly, compatibility of the polyisobutylene pressure-sensitive adhesive with the drug can be specifically increased. As a result, not only it becomes possible to increase blending amount of the drug but also bleed of the drug from the pressure-sensitive adhesive layer can be suppressed and, what is more, the pressure-sensitive adhesion characteristics sufficient from the practical point of view can be obtained.

*FIG. 1*

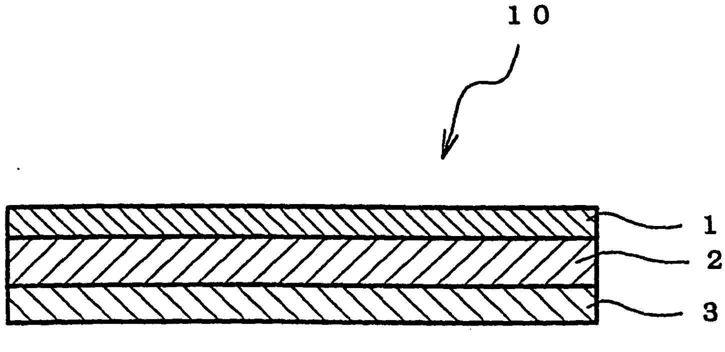




Printed by Jouve, 75001 PARIS (FR)

## Description

### TECHNICAL FIELD

The present invention relates to a percutaneous absorption adhesive pharmaceutical preparation for continuously administering a drug having a liquid quality at room temperature or around room temperature (wherein, free base of bisoprolol is excluded), into the body through the skin surface.

### BACKGROUND ART

As preparations for carrying out treatment or prevention of diseases by administering a drug into the living body, for example, there are percutaneous absorption pharmaceutical preparations which not only can avoid metabolism of a drug owing to a first pass effect of the liver and various side effects, but also can continuously administer the drug over a prolonged period of time. Among them, development of adhesive pharmaceutical preparations in which a drug is contained in a pressure-sensitive adhesive has been increasingly carried out because of the easy drug application work and the ability to strictly control the dose.

As the basic characteristics required for adhesive pharmaceutical preparations, pressure-sensitive adhesive characteristics may be mentioned from the practical point of view, in addition to the releasing property and stability of the drug. In developing adhesive pharmaceutical preparations, in order to satisfy these basic characteristics, designing of the adhesive pharmaceutical preparations is carried out by selecting most suitable pressure-sensitive adhesive and additive agent in accordance with the drug. As the pressure-sensitive adhesive, an acrylic pressure-sensitive adhesive and a rubber pressure-sensitive adhesive are mainly used, and from the viewpoint of drug stability in the pressure-sensitive adhesive, the rubber pressure-sensitive adhesive which does not have a functional group is generally advantageous than the acrylic pressure-sensitive adhesive. As the rubber pressure-sensitive adhesive, for example, polyisobutylene (PIB) pressure-sensitive adhesive, styrene-isoprene-styrene (SIS) pressure-sensitive adhesive and silicone pressure-sensitive adhesive may be mentioned, but since the SIS and silicone pressure-sensitive adhesives are difficult to be blended with sufficient fatty acid esters which can accelerate absorption of the drug, and the silicone pressure-sensitive adhesive is expensive, blending and selection of these components are limited and, as a result, degree of freedom of the designing of the adhesive pharmaceutical preparation becomes low. Accordingly, as the rubber pressure-sensitive adhesive, polyisobutylene pressure-sensitive adhesive (to be referred sometimes to as "PIB pressure-sensitive adhesive" hereinafter) is easy to use.

However, since the PIB pressure-sensitive adhesive has low polarity, it has a problem of being low in drug solubility. For the purpose of satisfying releasing quantity and persistency of a drug which are required for the adhesive pharmaceutical preparation, although it is desirable to blend the drug in an amount as large as possible, the amount of the drug is limited in the PIB pressure-sensitive adhesive. Even so, in the case of a drug which is solid at room temperature or around room temperature, it is possible to blend the drug in a large amount of its solubility or more by dispersing the solid drug in the pressure-sensitive adhesive. In such a case, since a part of the drug is dispersed in the pressure-sensitive adhesive in the form of crystals and the like, and the concentration of the drug dissolved in the pressure-sensitive adhesive is low, adhesion strength of the pressure-sensitive adhesive itself is not spoiled. That is, when a drug which is solid at room temperature or around room temperature is used, since it becomes possible to attain the blending of the drug in an amount necessary and sufficient for the treatment or prevention as well as the pressure-sensitive adhesive characteristics from the practical point of view, the poor drug solubility of the PIB pressure-sensitive adhesive does not become a large problem.

However, there are certain drugs which are liquid at room temperature or around room temperature. In the case of such drugs, blending of a drug and pressure-sensitive adhesive characteristics becomes incompatible when a large amount of the drug exceeding its solubility in a pressure-sensitive adhesive is blended in the pressure-sensitive adhesive. That is, a drug which cannot be sufficiently dissolved in a pressure-sensitive adhesive cannot be dispersed and present in the pressure-sensitive adhesive unlike the case of a solid drug, but flows in a PIB pressure-sensitive adhesive during the storage due to the fluidity of the drug itself to thereby exudes on the surface of the pressure-sensitive adhesive layer. This phenomenon of exuding is called bleed, and when the bleed occurs, surface of the pressure-sensitive adhesive layer is covered with the drug to inhibit contact of the pressure-sensitive adhesive with an adherend, so that the adhesion strength of the adhesive pharmaceutical preparation is considerably reduced. In addition, it not only reduce the adhesion strength to an adherend but also causes reduction of adhesiveness of the pressure-sensitive adhesive for the backing, namely reduction of anchorage property.

As the drug which is liquid at room temperature or around room temperature, for example, there are free base of emedastine, free base of gallopamil and the like. Although their salts are crystalline powder, a drug of a salt form becomes low in the percutaneous absorption. When these free bases are used for the purpose of improving percutaneous absorption, the percutaneous absorption is improved but it causes a problem of reducing stability of the drug. Accordingly,

if an adhesive pharmaceutical preparation which employs a PIB pressure-sensitive adhesive having excellent drug stability in a pressure-sensitive adhesive can be developed, its technical significance is large.

With the aim of improving reduction of pressure-sensitive adhesion characteristics in the case of blending a large amount of a liquid drug in the pressure-sensitive adhesive layer, Patent Reference 1 discloses a percutaneous composition which comprises one or more drugs wherein at least one of them has a low molecular weight and is liquid at room temperature or about room temperature, and a polymer matrix that contains one or more high shearing resistance polymers. It is described that this high shearing resistance polymer reduces the plasticizing effect of the low molecular weight drug and has the tackiness and shearing force sufficient for applying to human. In addition, it is described in its Examples that an acrylic pressure-sensitive adhesive or a pressure-sensitive adhesive containing a mixture of an acrylic pressure-sensitive adhesive and a silicone pressure-sensitive adhesive showed a stringiness suppressing effect. Although polyisobutylene is exemplified as the high shearing resistance polymer, the effect thereof is not verified in the Examples and the like, and PIB pressure-sensitive adhesives are not substantially examined.

In addition, according to a view of the present inventors, although it is theoretically possible to suppress stringiness of a pressure-sensitive adhesive in the case of increased blending amount of a liquid drug, by hardening the pressure-sensitive adhesive through the increase of molecular weight of a high shearing resistance polymer, increase of blending ratio of the high shearing resistance polymer and the like as described in the above-mentioned Patent Reference 1, when the pressure-sensitive adhesive is hardened, adhesiveness of the pressure-sensitive adhesive to an adherend is spoiled and adhesion strength of the adhesive pharmaceutical preparation is reduced. Since the acrylic pressure-sensitive adhesive has an originally high level of adhesion strength, reduction of the adhesion strength does not become a serious problem even when it contains a liquid drug, but in the case of the PIB pressure-sensitive adhesive which has low adhesion strength in comparison with the acrylic pressure-sensitive adhesive, when the PIB pressure-sensitive adhesive is hardened, its adhesion strength is sharply reduced so that assuring of its adhesion strength from the practical point of view becomes difficult. Accordingly, it is difficult to apply the method described in the Patent Reference 1 to the PIB pressure-sensitive adhesive, and it is the actual circumstances that an adhesive pharmaceutical preparation having sufficient pressure-sensitive adhesion characteristics owing to the combination of a drug which is liquid at room temperature or around room temperature and the PIB pressure-sensitive adhesive, has not been obtained yet.

Patent Reference 1: JP-A-2005-23088

## DISCLOSURE OF THE INVENTION

The present invention has been made with taking such actual circumstances into consideration, and the problem to be solved in the invention is, in developing an adhesive pharmaceutical preparation for percutaneously administering the drug which is liquid at room temperature or around room temperature into the living body, to provide an adhesive pharmaceutical preparation containing a polyisobutylene pressure-sensitive adhesive as the pressure-sensitive adhesive, which can suppress the bleed of the drug and has sufficient pressure-sensitive adhesion characteristics.

The present inventors have found that when a specified alcohol is contained in an pressure-sensitive adhesive layer containing a PIB pressure-sensitive adhesive and a drug which is liquid at room temperature or around room temperature, compatibility of a polyisobutylene pressure-sensitive adhesive with the drug is specifically increased and the above-mentioned problem can be solved as a result. When further examined in detail, the inventors have found that this is particularly effective for a drug in which its logPow and viscosity at 40°C are within specific ranges. The invention has been accomplished based on these findings.

That is, the invention has the following characteristics.

(1) An adhesive pharmaceutical preparation which comprises:

a backing; and
a pressure-sensitive adhesive layer laminated on one side of the backing, said pressure-sensitive adhesive layer containing a branched monoalcohol having from 12 to 28 carbon atoms, a drug which is liquid at room temperature or around room temperature (wherein free base of bisoprolol is excluded), and a polyisobutylene pressure-sensitive adhesive.

(2) The adhesive pharmaceutical preparation according to (1) above, wherein the drug has a logPow value of from -1.0 to 5.0 and a viscosity at 40°C of from 0.05 to 100,000 mPa·s.
(3) The adhesive pharmaceutical preparation according to (1) or (2) above, wherein the branched monoalcohol is a primary alcohol.
(4) The adhesive pharmaceutical preparation according to any one of (1) to (3) above, wherein the branched monoalcohol is a 2-alkyl-1-alkanol.
(5) The adhesive pharmaceutical preparation according to (4) above, wherein the number of carbons of the alkyl group at the 2-position of the 2-alkyl-1-alkanol is 2 or more.
(6) The adhesive pharmaceutical preparation according to any one of (1) to (5) above, wherein the branched monoalcohol is at least one kind selected from 2-hexyl-1-decanol, 2-octyl-1-decanol, 2-hexyl-1-dodecanol, 2-octyl-1-dodecanol and 2-decyl-1-tetradecanol.

According to the adhesive pharmaceutical preparation of the invention, the compatibility of a PIB pressure-sensitive adhesive with a drug which is liquid at room temperature or around room temperature can be specifically increased by containing a branched monoalcohol having from 12 to 28 carbon atoms as a solubilizing agent in the pressure-sensitive adhesive layer. As a result, not only it becomes possible to increase blending amount of drug but also bleed of drug from the pressure-sensitive adhesive layer can be suppressed and, what is more, the pressure-sensitive adhesion characteristics sufficient from the practical point of view can be obtained. Accordingly, an adhesive pharmaceutical preparation which can achieve compatibility of the pharmacological action with pressure-sensitive adhesion characteristics at high level can be provided.

## BRIEF DESCRIPTION OF THE DRAWINGS

Fig. 1 is a sectional view showing an embodiment of the adhesive pharmaceutical preparation of the invention.

Description of the Reference Numerals

1 Backing
2 Pressure-sensitive adhesive layer
3 Release liner
10 Adhesive pharmaceutical preparation

## BEST MODE FOR CARRYING OUT THE INVENTION

The following describes the invention in detail based on its suitable embodiments. In this connection, in the description of the drawing, overlapping descriptions are omitted by attaching the same reference numeral to the same element. In addition, for the sake of convenience of the illustration, dimensional ratios of the drawing do not necessarily coincide with the descriptions.
Fig. 1 is a sectional view showing a suitable embodiment of the adhesive pharmaceutical preparation of the invention. The adhesive pharmaceutical preparation 10 is provided with a backing 1, an pressure-sensitive adhesive layer 2 laminated on one side of the backing 1, and a release liner 3 laminated on the pressure-sensitive adhesive layer 2. The pressure-sensitive adhesive layer 2 contains a branched monoalcohol having from 12 to 28 carbon atoms, a polyisobutylene pressure-sensitive adhesive, and a drug which is liquid at room temperature or around room temperature (in which, free base of bisoprolol is excluded; to be referred simply to as "drug" hereinafter).
The branched monoalcohol to be contained in the pressure-sensitive adhesive layer functions as a solubilizing agent and, surprisingly, only a branched monoalcohol having from 12 to 28 (preferably from 16 to 24) carbon atoms can specifically improve compatibility of the PIB pressure-sensitive adhesive with the drug. As a result, not only it becomes possible to suppress the bleed of the drug but also the pressure-sensitive adhesion characteristics sufficient from a practical point of view can be ensured. In this connection, the branched monoalcohol can be used as one species alone or in combination of two or more species. In addition, the number of carbons means the total number of carbons of the carbon skeleton constituting the alcohol.
In order to improve compatibility of the drug with the PIB pressure-sensitive adhesive, it is considered that the compatibility can be improved to a certain degree when a solubilizing agent having a polarity between the drug and the PIB pressure-sensitive adhesive is used. Accordingly, it is considered that two or more compounds (such as esters and acids) other than the above-mentioned alcohol can be used, because their influence upon the compatibility is small even when the number of carbons, the kind or number of polar group or the binding position of polar group is slightly different in comparison with the above-mentioned alcohol. However, even when a fatty acid ester, a diester, an organic acid or the like having the same number of carbons and similar polarity is used instead of the above-mentioned alcohol, strangely, the effect of suppressing the bleed of the drug cannot be obtained at all or is very small even when it is obtained. In

addition, in the case of a monoalcohol which has the same number of carbons and its carbon skeleton is linear chain , since its bulk height is low in comparison with the above-mentioned branched alcohol, intermolecular interaction of the alcohol becomes strong to thereby lower the fluidity of the alcohol itself in some cases. Thus, the fluidity and deformability of the pressure-sensitive adhesive are reduced and adhesion strength of the pressure-sensitive adhesive is also lowered in some cases. In addition, even in the case of a branched monoalcohol, when the number of carbons is smaller than 12, the hydrophobicity based on the carbon skeleton becomes small and the compatibility of drug with the PIB pressure-sensitive adhesive is rapidly reduced. On the other hand, when the number of carbons is larger than 28, the hydrophobicity becomes so large that the compatibility of drug with the PIB pressure-sensitive adhesive is reduced.

As the branched monoalcohol having from 12 to 28 carbon atoms, a primary alcohol is desirable because it is apt to interact with the drug due to the aptness of its hydroxyl group to be exposed to the surface of the alcohol molecule and, as a result, solubility of the drug is significantly improved. Particularly, 2-alkyl-1-alkanol having an excellent balance between its hydroxyl group and carbon skeleton is more preferable. The alcohol has such a structure that one carbon chain branching base point is present and two long carbon chains and one short carbon chain elongate from the base point, in which a hydroxyl group is linked to the tip of the short carbon chain. Owing to the possession of such a structure, the whole alcohol molecules become high in bulk and intermolecular interaction between alcohol molecules is weakened so that fluidity of the alcohol molecules is increased. In addition, since the two long carbon chains efficiently interact as a hydrophobic moiety with the PIB molecule, compatibility of the alcohol molecule with the PIB molecule is improved. Further, since the hydroxyl group of the alcohol molecule does not hide in the inner part of the alcohol molecule but is modestly exposed to the surface of the alcohol molecule, the hydroxyl group can interact with the drug, whereby the compatibility of the alcohol with the drug is improved. The branching effect of carbon chains of the alcohol molecule and the effect of exposure of hydroxyl group were revealed from the comparison of 2-hexyl-1-decanol with hexadecane-8-ol, as monoalcohols having similar molecular structures (cf. Inventive Example 2 and Comparative Example 5).

As the above-mentioned alcohol, those in which the number of carbons of the alkyl group at the 2-position is 2 or more (preferably from 4 to 12, more preferably from 6 to 10) are particularly suitably used. Illustratively, 2-butyl-1-octanol, 2-ethyl-1-decanol, 2-propyl-1-decanol, 2-hexyl-1-octanol, 2-hexyl-1-decanol, 5,7,7-trimethyl-2-(1,3,3-trimethylbutyl)-1-octanol, 2-heptyl-1-undecanol, 2-ethyl-1-hexadecanol, 2-hexyl-1-decanol, 2-hexyl-1-dodecanol, 2-octyl-1-decanol, 2-octyl-1-dodecanol, 2-decyl-1-tetradecanol and 2-dodecyl-1-hexadecanol can be exemplified. Among them, 2-octyl-1-dodecanol, 2-hexyl-1-decanol, 2-octyl-1-decanol, 2-hexyl-1-dodecanol and 2-decyl-1-tetradecanol are more preferable.

The content of the above-mentioned alcohol based on the total weight of the pressure-sensitive adhesive layer can be optionally selected depending on the content of drug and the like and therefore is not particularly limited, but it is generally from 0.1 to 40% by weight, preferably from 0.5 to 35% by weight, more preferably from 0.5 to 30% by weight, most preferably from 0.5 to 20% by weight. When the content is smaller than 0.1 % by weight, it causes a tendency that the above-mentioned effect cannot be fully obtained while, on the other hand, when the content is larger than 40% by weight, it causes a tendency that the cohesive force and adhesion strength of the whole pressure-sensitive adhesive layer are reduced. The invention can be advantageously carried out when the above-mentioned alcohol content is from 0.1 to 40% by weight, from the viewpoint that the drug bleed can be efficiently suppressed in comparison with the case of using other organic liquid components such as a fatty acid alkyl ester.

The drug to be contained in the pressure-sensitive adhesive layer is not particularly limited so long as it is a drug which is liquid at room temperature or around room temperature, but a drug having a partition coefficient (logPow) of from - 1.0 to 5.0 and a viscosity at 40°C of from 0.05 to 100,000 mPa·s is suitable. Illustratively, the emedastine free base represented by the following formula (1), crotamiton represented by the following formula (2), gallopamil free base represented by the following formula (3) and the like can be exemplified.

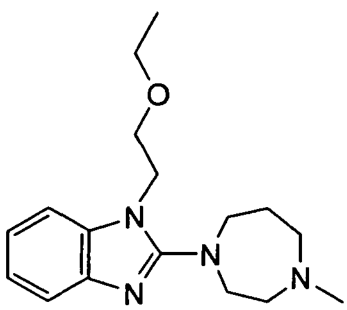

(1)

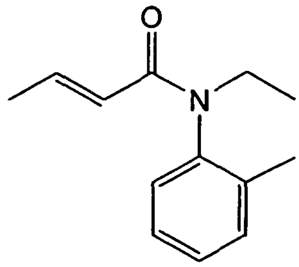

(2)

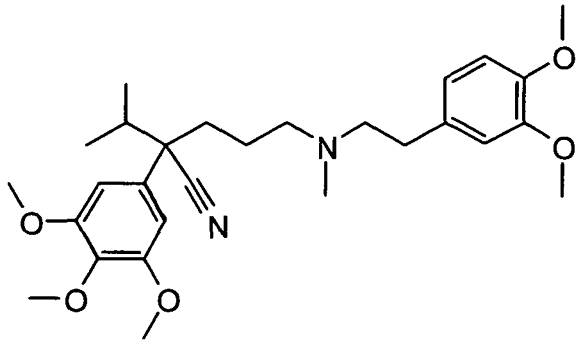

(3)

In this case, the "drug which is liquid at room temperature or around room temperature" means a drug which is liquid at a temperature where the adhesive pharmaceutical preparation after its production is generally preserved, namely any temperature within a temperature range of from 1 to 40°C. Within such a temperature range, the drug has such a degree of fluidity that it can cause bleeding. Viscosity of the drug is measured using a type E viscometer while keeping the drug at a predetermined temperature. According to the invention, when the viscosity of a drug at a predetermined temperature is from 0.05 to 100,000 mPa·s, the drug is liquid at the predetermined temperature. In addition, the "logPow" is an index which expresses hydrophilic property or hydrophobicity of the drug, and is a value measured on individual drugs in accordance with the method described in "OECD GUIDELINE FOR THE TESTING OF CHEMICALS 107, Adopted by the Council on $27^{th}$ July 1995, Partition Coefficient (n-octanol/water), Shake Flask Method)", and the base of logarithm of 10gPow is 10.

The "logPow" is obtained by a calculation after partitioning a drug into water layer and n-octanol layer and determining the drug concentration of each layer by an appropriate method, in accordance with the method described in the above-mentioned guideline. The quantitative determination of drugs is not particularly limited so long as it is a method in which its determinability was verified, for example, by verifying linearity of its calibration curve, but in general, an absorptiometry, a gas chromatography or a high performance liquid chromatography is used. In the case of the measurement on a drug which does not have UV absorption, a titration method and the like other methods may be used when their determinability is verified. In addition, when the drug is a dissociating substance, the logPow is measured while suppressing its dissociation in accordance with the guideline. That is, the logPow is measured using a buffer of appropriate pH as the water layer under a condition of a free acid or free base. When the drug is an acidic drug, a buffer having a pH value which is lower by a factor of 1 than the pKa value of the drug is used. When the drug is a basic drug, a buffer having a pH value which is higher by a factor of 1 than the pKa value of the drug is used.

In this connection, although the measurement range of logPow is generally set from -2 to 4 according to this guideline, the measurement range of logPow can be set to -2 to 5 by increasing the determination sensitivity by making use of high sensitivity analytical methods such as a high performance liquid chromatography.

When the logPow is smaller than -1.0, hydrophilic property of the drug is too high and its compatibility with the PIB pressure-sensitive adhesive therefore is low, so that there is a case in which the bleed suppressing effect cannot be obtained sufficiently even when the above-mentioned alcohol is added. On the other hand, when the logPow is larger than 5.0, there is a case in which the bleed suppressing effect and adhesiveness cannot be obtained sufficiently. In this connection, according to a finding by the present inventors, there is a remarkable difference in the bleed suppressing effect depending on the presence or absence of the addition of the above-mentioned alcohol in the case of a drug having a logPow value of from -1.0 to 5.0 (preferably from 0.0 to 5.0), so that the invention has a technically large significance from this point of view.

In addition, the viscosity of a drug at 40°C is measured using a type E viscometer while heating the drug at 40°C. The viscosity at 40°C is generally from 0.05 to 100,000 mPa·s, preferably from 0.5 to 50,000 mPa·s. In the case

that the viscosity is smaller than 0.05 mPa·s, when it is blended with a pressure-sensitive adhesive solution and it is then spread and dried on a base material, there is a tendency that a drug is apt to vaporize from the pressure-sensitive adhesive layer due to the excessively high fluidity. On the other hand, when the viscosity at 40°C is larger than 100,000 mPa·s, there is a tendency that weighing operation of the drug cannot be carried out precisely due to the excessively high viscosity.

Although the content of the drug in the pressure-sensitive adhesive layer is not particularly limited and can be optionally selected based on the releasing ability and solubility of the drug and the above-mentioned effect which is obtained depending on the kind and addition amount of the alcohol, it is preferably from 0.1 to 40% by weight, more preferably from 0.5 to 40% by weight, further preferably from 0.5 to 35% by weight. When the content of the drug is less than 0.1 % by-weight, its bleeding can be suppressed owing to the sufficiently low concentration of the drug, but there is a tendency that the effect owing to the addition of the above-mentioned alcohol cannot be sufficiently expressed. On the other hand, when the content of the drug exceeds 40% by weight, since the concentration of the drug is too high, there is a tendency that the bleed suppressing effect owing to the addition of the above-mentioned alcohol cannot be sufficiently expressed. When the content of the drug is from 0.1 to 40% by weight, a remarkable difference in the bleed suppressing effect is recognized depending on the presence or absence of the addition of the above-mentioned alcohol, so that the invention has a technically large significance from this point of view.

In addition, the PIB pressure-sensitive adhesive to be used in the adhesive pharmaceutical preparation of the invention is not particularly limited so long as it essentially contains polyisobutylene and it has appropriate tackiness and cohesiveness as the pressure-sensitive adhesive by itself, and one species alone or a combination of two or more species may be used. In addition, when polyisobutylene is contained in one species alone, molecular weight of the polyisobutylene is not particularly limited, but its viscosity average molecular weight is preferably from 40,000 to 5,500,000, more preferably from 45,000 to 5,000,000. When the viscosity average molecular weight is less than 40,000, there is a possibility that it becomes difficult to provide internal cohesive force necessary for the pressure-sensitive adhesive layer while, when it exceeds 5,500,000, there is a possibility that skin adhesiveness and tack of the pressure-sensitive adhesive layer are deteriorated.

From the viewpoint of easily achieving appropriate flexibility of the pressure-sensitive adhesive layer as well as its irritation to the skin, it is desirable to contain at least two species of polyisobutylene having different molecular weights. As such polyisobutylene, those which are constituted of a combination of a first polyisobutylene and a second polyisobutylene having a lower molecular weight than that of the first polyisobutylene is preferable, and two or more species of polyisobutylene having different molecular weights can be combined when they are within the respective molecular weight distribution ranges. In this case, the "at least two species of polyisobutylene having different molecular weights" according to the present specification means a polyisobutylene which has molecular weight distribution peaks measured by a gel permeation chromatography (GPC) in at least two independent areas.

When the polyisobutylene is constituted of two species of polyisobutylene, molecular weight of each polyisobutylene is not particularly limited, but it is desirable for obtaining excellent tackiness that the viscosity average molecular weight of the first polyisobutylene is preferably from 1,800,000 to 5,500,000, more preferably from 2,000,000 to 5,000,000, and the viscosity average molecular weight of the second polyisobutylene is preferably from 40,000 to 85,000, more preferably from 45,000 to 65,000. In this case, when the viscosity average molecular weight of the first polyisobutylene is less than 1,800,000, there is a tendency that it becomes difficult to obtain the internal cohesive force necessary for the pressure-sensitive adhesive layer while, when it exceeds 5,500,000, there is a tendency that skin adhesiveness and tack of the pressure-sensitive adhesive layer are reduced. In addition, when the viscosity average molecular weight of the second polyisobutylene is less than 40,000, there is a possibility that a sticky feeling is expressed in the pressure-sensitive adhesive layer to thereby stain the skin surface while, when it exceeds 85,000, there is a tendency that skin adhesiveness and tack of the pressure-sensitive adhesive layer are reduced.

In this connection, the viscosity average molecular weight according to the present specification means a value obtained by calculating Staudinger's index ($J_0$) by Suhulz-Blaschke formula from the capillary flow time of Ubbelohde's viscometer at 200°C, and calculating it according to the following formula using this Jo value.

$$J_0 = \eta_{SP}/c(1 + 0.31\ \eta_{SP})\mathrm{cm}^3/\mathrm{g}\ \text{(Suhulz-Blaschke formula)}$$

$$\eta_{SP} = t/t_0 - 1$$

t: flow time of the solution (by Hagenbach-couette correction formula)
to: flow time of the solvent (by Hagenbach-couette correction formula)

c: concentration of the solution ($g/cm^2$)

$$J_0 = 3.06 \times 10^{-2} Mv^{0.65}$$

Mv: viscosity average molecular weight

When the polyisobutylene is constituted of two species of polyisobutylene having different molecular weights, blending ratio of the first polyisobutylene to the second polyisobutylene in terms of weight ratio is preferably from 1/0.1 to 1/3, more preferably from 1/0.1 to 1/2.5, further preferably from 1/0.3 to 1/2. Of these two species of polyisobutylene, when blending ratio of the second polyisobutylene is less than the lower limit, there is a tendency that reduction of the skin adhesion strength of the pressure-sensitive adhesive layer becomes large while, when it exceeds the above-mentioned upper limit, there is a tendency that reduction of the internal cohesive force of the pressure-sensitive adhesive layer becomes large.

The total polyisobutylene content based on total weight of the pressure-sensitive adhesive layer is preferably from 15 to 70% by weight, more preferably from 15 to 60% by weight. When the polyisobutylene content is less than 15% by weight, there is a possibility that it becomes difficult to provide internal cohesive force necessary for the pressure-sensitive adhesive layer while, when it exceeds 70% by weight, there is a possibility that skin adhesiveness and tack of the pressure-sensitive adhesive layer are reduced.

According to the invention, a tackifier may be optionally contained. As the tackifier, those which are conventionally known in the field of adhesive pharmaceutical preparations can be optionally selected and used, and examples thereof include petroleum resins, terpene resins, rosin resins, coumarone-indene resins, styrene resins, and alicyclic saturated hydrocarbon resins. Among them, alicyclic saturated hydrocarbon resins are preferable because of its excellent drug storage stability. In addition, from the viewpoint of obtaining good tack, a tackifier having a softening point of preferably from 90 to 150°C, more preferably from 95 to 145°C, is used. For example, in the case of alicyclic saturated hydrocarbon resins, tack and cohesive force of the pressure-sensitive adhesive layer tend to be lowered when the softening point is less than 90°C while, when it exceeds 150°C, there is a tendency that the pressure-sensitive adhesive layer becomes hard to thereby deteriorate the skin adhesiveness. Accordingly, the skin adhesiveness, cohesive force and drug stability may be improved when the adhesive pharmaceutical preparation is prepared by optionally selecting the kind and softening point of the tackifier. In this connection, the softening point according to the present specification means a value measured in accordance with the ring and ball method (JIS K 6863).

As the alicyclic saturated hydrocarbon resin, for example, Alcon P-100, Alcon P-115, Alcon P-125 and Alcon P-140 (trade names, mfd. by Arakawa Chemical Industries) may be mentioned as articles on the market.

The tackifier can be used as one species or in combination of two or more species, and when used in combination of two or more species, for example, resins having different kinds and softening points may be combined.

The content of the tackifier based on the total weight of the pressure-sensitive adhesive layer is preferably from 15 to 55% by weight, more preferably from 20 to 50% by weight. When the content of the tackifier is less than 15% by weight, tack is poor in some cases while, when it exceeds 55% by weight, there is a tendency that the pressure-sensitive adhesive layer becomes hard to thereby deteriorate the skin adhesiveness.

In addition, according to the invention, from viewpoint of the absorption acceleration of drug and the like, an organic liquid component other than the above-mentioned alcohol and drug may also be optionally contained. The organic liquid component is not particularly limited so long as it is compatible with the polyisobutylene and tackifier, and a fatty acid alkyl ester may for example be mentioned.

Examples of the fatty acid alkyl ester include a fatty acid alkyl ester obtained from a higher fatty acid having from 12 to 16, preferably from 12 to 14 carbon atoms and a lower monoalcohol having from 1 to 4 carbon atoms . The higher fatty acid is preferably lauric acid (C12), myristic acid (C14) or palmitic acid (C16), and more preferably myristic acid. As the monoalcohol, methyl alcohol, ethyl alcohol, propyl alcohol, isopropyl alcohol, butyl alcohol and the like may be exemplified, of which isopropyl alcohol is preferred. Accordingly, most preferred fatty acid alkyl ester is isopropyl myristate.

The organic liquid component may be used one species alone or as a combination of two or more species. The content of the organic liquid component based on the total weight of the pressure-sensitive adhesive layer is preferably from 1 to 40% by weight, more preferably from 3 to 25% by weight. When the organic liquid component content is less than 1% by weight, there is a tendency that absorption acceleration and the like effects are not sufficiently exerted while, when it exceeds 40% by weight, there is a tendency that the adhesion strength and cohesive force of the whole pressure-sensitive adhesive are reduced.

The backing is not particularly limited, but those being substantially practically impermeable to drugs is preferably used; that is, the backing causing no decrease of the content because drug as active ingredient and additives and the

like are lost from the rear side across the backing. As the backing, for example, a single film of polyester, nylon, polyvinylidene chloride, polyethylene, polypropylene, polyvinyl chloride, ethylene-ethyl acrylate copolymer, polytetrafluoroethylene, ionomer resin, metal foil or the like or a laminate film thereof and the like may be used. Among these, in order to further improve adhesiveness (anchorage property) of the backing with the pressure-sensitive adhesive layer, it is preferable to make the backing into a laminate film of a non-porous plastic film of the above-mentioned material and a porous film. In this case, it is desirable to form the pressure-sensitive adhesive layer on the porous film side.

As such a porous film, those which improve anchorage property with the pressure-sensitive adhesive layer are employed, and illustratively, paper, woven fabric, non-woven fabric, knitting, a mechanically punching-treated sheet and the like can be exemplified. Among these, paper, woven fabric and non-woven fabric are particularly preferred from the viewpoint of easiness of handling and the like. A porous film having a thickness of from 10 to 200 μm is employed in view of the improvement of anchorage property, flexibility of the whole adhesive pharmaceutical preparation, and easiness of sticking. In a case of relatively thin adhesive pharmaceutical preparation such as plaster type or adhesive tape type, those which have a thickness of from 10 to 100 μm are employed.

Further, in the case where a woven fabric or a non-woven fabric is used as the porous film, its basis weight is preferably from 5 to 30 $g/m^2$, and more preferably from 6 to 15 $g/m^2$. As the most favorable backing, a laminated film of a polyester film (preferably a polyethylene terephthalate film) having a thickness of from 1.5 to 6 μm and a non-woven fabric made of a polyester (preferably polyethylene terephthalate) having a basis weight of from 6 to 12 $g/m^2$ is exemplified.

In the adhesive pharmaceutical preparation of the invention, for the purpose of protecting the pressure-sensitive adhesive surface of the pressure-sensitive adhesive layer until the time of use, it is desirable that a release liner is laminated on the pressure-sensitive adhesive surface. The release liner is not particularly limited so far as it can be subjected to a releasing treatment and is able to assure a sufficiently light peeling force, and the examples of the release liner include films such as polyesters, polyvinyl chloride, polyvinylidene chloride and polyethylene terephthalate, papers such as high-quality papers and glassine papers or film of polyolefin laminated with high quality paper or glassine paper, to which releasing treatment is made by applying silicone resin or fluororesin on the surface contacting with the pressure-sensitive adhesive layer. The thickness of the release liner is preferably from 10 to 200 μm, and more preferably from 25 to 100 μm.

As the release liner, one made of a polyester (especially polyethylene terephthalate) resin is preferable from the standpoints of barrier properties, costs and the like. Furthermore, in that case, one having a thickness of from about 25 to 100 μm is preferable from the standpoint of easiness of handling.

The adhesive pharmaceutical preparation of the invention can be produced, for example, by dissolving a PIB pressure-sensitive adhesive containing two species of polyisobutylene having different molecular weights and a tackifier, the above-mentioned monoalcohol and the drug into an appropriate solvent such as toluene, forming a pressure-sensitive adhesive layer by applying and drying the thus obtained solution of composition for forming the pressure-sensitive adhesive layer on a release liner, and then laminating a backing on the pressure-sensitive adhesive layer. Alternatively, it can also be produced, for example, by directly applying the above-mentioned solution of the composition for forming the pressure-sensitive adhesive layer on a backing and drying it, thereby forming the pressure-sensitive adhesive layer on the backing. It is difficult in some cases to uniformly dry the solution of the composition for forming the pressure-sensitive adhesive layer when this is thickly applied in one portion, so that it is also possible to repeat the applying operation twice or more to give a pressure-sensitive adhesive layer having sufficient thickness . In this connection, thickness of the pressure-sensitive adhesive layer is generally from 10 to 300 μm, preferably from 20 to 250 μm. In addition, shape of the adhesive pharmaceutical preparation is not particularly limited, and for example, it may be a tape shape, a sheet shape or the like.

It is preferable that the adhesive pharmaceutical preparation of the invention is preserved or transported in a form of sealed package just before use. Packaging may be made, for example, by packing a single sheet of adhesive pharmaceutical preparation or several sheets of piled adhesive pharmaceutical preparations with a wrapping material and then tightly closing the periphery with a heat seal. The wrapping material includes, for example, a sheet-form or film-form material, for which there is no particular limitation. In this case, a material allowing heat sealing is desirous in view of easiness of packaging or air-tightness. Such a packaging material includes, specifically and preferably, those using a heat-sealable plastic sheet such as polyethylene, ionomer resin, ethylene-vinyl acetate copolymer, ethylene-vinyl alcohol copolymer, polyacrylonitrile copolymer, polyvinyl alcohol copolymer, and the like. In particular, in order to prevent the contamination or oxidation of an active ingredient bisoprolol contained in the adhesive pharmaceutical preparation by contact with ambient air, it is preferred to use a laminated gas-impermeable film such as polyester film or metal foil. The packaging material is used in thickness of 10 to 200μm. It is more preferable to use a high barrier polyacrylonitrile copolymer as the most inner layer of the above packaging material.

Further, it is appropriate to think out a packaging form formed by embossing of the packaging material, dry edge processing (slightly enlarging the above liner portion compared to the adhesive pharmaceutical preparation) or blister molding processing (making the contact area small), since it is feared that handling of the package such as taking-out from the package becomes worse when the pressure-sensitive adhesive ingredient is flowed out from the side of

the adhesive pharmaceutical preparation.

The adhesive pharmaceutical preparation of the invention may be taken out from the package, for example by tearing the above package, just before use, and the release liner is peeled off, and the exposed pressure-sensitive adhesive surface is then applied to the skin.

In addition, although it varies depending on the age, body weight, symptoms and the like of the patient, the adhesive pharmaceutical preparation of the invention is applied to the skin surface generally about once a day or two days in the case of adult.

EXAMPLES

The following describes the invention further illustratively with reference to examples, but these examples do not limit the invention. In this connection, the abbreviations to be used in the examples and the like are as follows.

PIB: PIB pressure-sensitive adhesive (composition: B200/6H/P140 = 34/26/40)
B200: Oppanol (R) B200 (mfd. by BASF) polyisobutylene, viscosity average molecular weight of 4,000,000
6H: HIMOL 6H (mfd. by Nippon Petrochemicals) polyisobutylene, viscosity average molecular weight of 60,000
P 140: ARKON (R) P 140 (mfd. by Arakawa Chemical Industries) tackifier, alicyclic saturated hydrocarbon resin, softening point of 140°C
IPM: LISONOL 18SP (mfd. by Kokyu Alcohol Kogyo) a mixture of 2-octyl-1-decanol/2-hexyl-1-dodecanol = 1/1

(Inventive Examples 1 to 6 and Comparative Examples 1 to 12)

A viscous solution was prepared by dissolving each composition for forming pressure-sensitive adhesive layer formulated in accordance with Table 1 in toluene. The solution thus obtained was coated on a silicone release treatment-applied liner (75 μm) made of polyethylene terephthalate (PET) to yield a thickness of 40 μm after drying, and a pressure-sensitive adhesive layer was formed by drying the same in a hot air circulation dryer to thereby remove toluene. Subsequently, a PET film having a thickness of 25 μm as a backing was applied on the pressure-sensitive adhesive layer to obtain a sheet-shaped adhesive pharmaceutical preparation.

Table 1

| | Formulation of composition for forming pressure-sensitive adhesive layer | | | | |
|---|---|---|---|---|---|
| | Drug (LogPow) | Drug content (wt%) | Solubilizing agent | Dissolving agent content (wt%) | Other components (wt%) |
| Inv. Ex. 1 | Emedastine free base (2.4) | 7.5 | 2-Octyl-1-dodecanol | 15 | PIB (77.5) |
| Inv. Ex. 2 | Emedastine free base (2.4) | 7.5 | 2-Hexyl-1-decanol | 15 | PIB (77.5) |
| Inv. Ex. 3 | Emedastine free base (2.4) | 7.5 | 2-Decyl-1-tetradecanol | 15 | PIB (77.5) |
| Inv. Ex. 4 | Emedastine free base (2.4) | 7.5 | 18 SP | 15 | PIB (77.5) |
| Inv. Ex. 5 | Crotamiton (3.1) | 12.5 | 2-Octyl-1-dodecanol | 15 | PIB (72.5) |
| Inv. Ex. 6 | Gallopamil free base (4.8) | 4.0 | 2-Octyl-1-dodecanol | 15 | PIB (81) |
| Comp. Ex. 1 | Emedastine free base (2.4) | 7.5 | IPM | 15 | PIB (77.5) |
| Comp. Ex. 2 | Emedastine free base (2.4) | 7.5 | Isostearic acid | 15 | PIB (77.5) |
| Comp. Ex. 3 | Emedastine free base (2.4) | 7.5 | Propylene glycol dicaprylate | 15 | PIB (77.5) |

(continued)

| | Formulation of composition for forming pressure-sensitive adhesive layer | | | | |
|---|---|---|---|---|---|
| | Drug (LogPow) | Drug content (wt%) | Solubilizing agent | Dissolving agent content (wt%) | Other components (wt%) |
| Comp. Ex. 4 | Emedastine free base (2.4) | 7.5 | Eicosan-1-ol | 15 | PIB (77.5) |
| Comp. Ex. 5 | Emedastine free base (2.4) | 7.5 | Hexadecan-8-ol | 15 | PIB (77.5) |
| Comp. Ex. 6 | Emedastine free base (2.4) | 7.5 | 2-Ethyl-1-hexanol | 15 | PIB (77.5) |
| Comp. Ex. 7 | Crotamiton (3.1) | 12.5 | IPM | 15 | PIB (72.5) |
| Comp. Ex. 8 | Gallopamil free base (4.8) | 4.0 | IPM | 15 | PIB (81) |
| Comp. Ex. 9 | Glycerol (-1.1) | 10 | 2-Octyl-1-dodecanol | 10 | PIB (75)<br>IPM (5) |
| Comp. Ex. 10 | Glycerol (-1.1) | 10 | IPM | 15 | PIB (75) |
| Comp. Ex. 11 | Terbinafine free base (>5.0) | 25 | 2-Octyl-1-dodecanol | 15 | PIB (60) |
| Comp. Ex. 12 | Terbinafine free base (>5.0) | 25 | IPM | 15 | PIB (60) |

The following tests were carried out using the respective adhesive pharmaceutical preparations obtained in Inventive Examples 1 to 6 and Comparative Examples 1 to 12.

1. Bleed resistance

Whether or not a liquid substance was adhered to the liner when the liner was peeled off from the adhesive pharmaceutical preparation was visually observed and evaluated by the following criteria. The evaluation results are shown in Table 2.

O: A liquid substance was not adhered to the liner.
Δ: A liquid substance was slightly adhered to the liner.
X: A liquid substance was massively adhered to the liner.

2. Anchorage property

Whether or not the pressure-sensitive adhesive layer was anchored to the backing side when the liner was peeled off from the adhesive pharmaceutical preparation (liner release operation) was evaluated. In addition, whether or not the pressure-sensitive adhesive layer was anchored to the backing side when an adhesive pharmaceutical preparation was applied to a phenol resin plate and the adhesive pharmaceutical preparation was peeled off therefrom was evaluated (adhesiveness test). In this connection, the anchorage property was evaluated based on the following criteria. The evaluation results are shown in Table 2.

O: The pressure-sensitive adhesive layer was anchored to the backing in accordance with both of the liner release operation and adhesiveness test.
Δ: The pressure-sensitive adhesive layer was anchored to the backing in accordance with the liner release operation, but the pressure-sensitive adhesive layer was not anchored to the backing in accordance with the adhesiveness test.
X: The pressure-sensitive adhesive layer was not anchored to the backing in accordance with the liner release operation.

3. Adhesion feeling

With respect to the adhesion feeling when the pressure-sensitive adhesive layer exposed after peeling of the liner was touched with a finger, sensory evaluation was carried out based on the following criteria. The evaluation results are shown in Table 2.

O: The adhesion feeling was sufficiently strong.
Δ: The adhesion feeling was slightly weak.
X: The adhesion feeling was weak.

4. Adhesion strength measurement

Adhesion strength (peeling strength) was measured by applying each belt-shaped sample cut into a width of 24 mm to a phenol resin plate, closely adhering it by one reciprocation of a roller having a load of 850 g, and then peeling it off to the 180 degree direction at a rate of 300 mm/minute. The measured results are shown in Table 2.

5. Measurement of viscosity at 40°C

Viscosity of drugs at 40°C was measured using a type E viscometer (type E viscometer mfd. By Tokyo Keiki, VISCONIC Type ED). The measured results are shown in Table 3.

6. Measurement of logPow

LogPow was measured in accordance with the "OECD GUIDELINE FOR THE TESTING OF CHEMICALS 107, Adopted by the Council on 27$^{th}$ July 1995, Partition Coefficient (n-Octanol/water), Shake Flask Method)". Water layers, determination methods and results of the LogPow measurement at the time of the measurement of LogPow are shown in Table 3.

Table 2

| | Bleed resistance | Anchorage property | Adhesion feeling | Adhesion strength (N / 24 mm) |
|---|---|---|---|---|
| Inv. Ex. 1 | O | O | O | 7.7 |
| Inv. Ex. 2 | O | O | O | 1.8 |
| Inv. Ex. 3 | O | O | O | 6.6 |
| Inv. Ex. 4 | O | O | O | 1.3 |
| Inv. Ex. 5 | O | O | O | 0.22 |
| Inv. Ex. 6 | O | O | O | 0.72 |
| Comp. Ex. 1 | X | Δ | X | Not measurable |
| Comp. Ex. 2 | X | Δ | X | Not measurable |
| Comp. Ex. 3 | X | X | X | Not measurable |
| Comp. Ex. 4 | Δ | Δ | X | Not measurable |
| Comp. Ex. 5 | Δ | O | O | 0.2 |
| Comp. Ex. 6 | X | X | X | Not measurable |
| Comp. Ex. 7 | O | O | X | 0.05 |
| Comp. Ex. 8 | Δ | Δ | X | Not measurable |
| Comp. Ex. 9 | Δ | O | X | 8.2 |
| Comp. Ex. 10 | Δ | O | X | 9.4 |
| Comp. Ex. 11 | Δ | O | O | 0.25 |
| Comp. Ex. 12 | Δ | O | Δ | 0.12 |

Table 3

| Drug | Water layer | Determination method | LogPow | Viscosity (40°C, mPa·s) | Molecular weight |
|---|---|---|---|---|---|
| Emedastine free base | pH 10 Buffer | HPLC method | 2.4 | 280 | 302 |
| Crotamiton | Distilled water | HPLC method | 3.1 | 16 | 203 |
| Gallopamil free base | pH 10 buffer | HPLC method | 4.8 | 4500 | 485 |
| Glycerol | Distilled water | Titration method | -1.1 | 270 | 92 |
| Terbinafine free base | pH 9.2 Buffer | HPLC method | >5.0 | 280 | 291 |

<Results>

Regarding Inventive Examples 1 to 4 and Comparative Examples 1 to 6

In the adhesive pharmaceutical preparations of Inventive Examples 1 to 4, which contain, as the drug, emedastine free base having a viscosity (40°C) of 280 mPa·s and a logPow value of 2.4, and contain 2-octyl-1-dodecanol, 2-hexyl-1-decanol, 2-decyl-1-tetradecanol or a mixture of 2-octyl-1-decanol and 2-hexyl-1-dodecanol, bleed was not observed, and their anchorage property and adhesion feeling were both good and the adhesion strength was also strong in comparison with the comparative examples.

On the other hand, in the adhesive pharmaceutical preparations of Comparative Example 1 to 4 and 6, which respectively contain isopropyl myristate (ester), isostearic acid (acid), propylene glycol dicaprylate (diester), eicosan-1-ol (straight chain alcohol) and 2-ethyl-1-hexanol (2-alkyl-1-alkanol having 8 carbon atoms), bleed was found, adhesion was weak, anchorage property was insufficient and, what is more, adhesion strength could not be measured because of the insufficient anchorage property.

In addition, in the adhesive pharmaceutical preparation of Comparative Example 5, which contains hexadecane-8-ol that has a structure similar to 2-hexyl-1-decanol but is a straight chain secondary alcohol, although it possessed of an appropriate anchorage property, it was inferior in the adhesion strength in comparison with the adhesive pharmaceutical preparations of Inventive Examples 1 to 4.

Regarding Inventive Example 5 and Comparative Example 7

In the adhesive pharmaceutical preparation of Inventive Example 5, which contains crotamiton that has a viscosity (40°C) of 16 mPa·s and a logPow value of 3.1 and also contains 2-octyl-1-dodecanol, bleed was not observed, its anchorage property and adhesion feeling were both good, and it possessed of sufficient adhesion strength.

On the other hand, in the case of the adhesive pharmaceutical preparation of Comparative Example 7, which contains isopropyl myristate, its adhesion strength was insufficient.

Regarding Inventive Example 6 and Comparative Example 8

In the case of the adhesive pharmaceutical preparation of Inventive Example 6, which contains gallopamil that has a viscosity (40°C) of 4,500 mPa·s and a logPow value of 4.8 and to which 2-octyl-1-dodecanol was added, bleed was not observed and its anchorage property and adhesion feeling were both good.

On the other hand, in the case of the adhesive pharmaceutical preparation of Comparative Example 8, which contains isopropyl myristate, bleed was found, adhesion feeling and anchorage property were weak, and the adhesion strength could not be measured because of the insufficient anchoring.

Regarding Comparative Examples 9 and 10

In the case of the adhesive pharmaceutical preparations of Comparative Examples 9 and 10, which contain glycerol that has a viscosity (40°C) of 270 mPa·s and a logPow value of -1.1, glycerol bled like spots because it does not show compatibility with the PIB pressure-sensitive adhesive due to its high hydrophilic property. In addition, there

was an adhesion feeling for a moment when the pressure-sensitive adhesive side was touched by a finger, but the touched finger easily slipped due to the bleed, the bled liquid matter spread all over the finger, and the adhesion feeling was entirely lost in that instant. In the adhesion strength test, portions which were free from the spotty bleed showed higher values of the adhesion strength, but were not practical because of the occurrence of the above-mentioned phenomenon. The bleed suppressing effect was not obtained even when 2-octyl-1-dodecanol was added.

Regarding Comparative Examples 11 and 12

In the case of the adhesive pharmaceutical preparations of Comparative Examples 11 and 12, which contain terbinafine that has a viscosity (40°C) of 280 mPa·s and a logPow value of larger than 5.0, bleed did not occur when the drug content was 20% by weight because of the originally good compatibility with the PIB pressure-sensitive adhesive, but the bleed was generated a little when the drug content was set to 25% by weight. In addition, there was no bleed suppressing effect even when isopropyl myristate was changed to 2-octyl-1-dodecanol.

While the invention has been described in detail and with reference to specific embodiments thereof, it will be apparent to one skilled in the art that various changes and modifications can be made therein without departing from the spirit and scope thereof.

The present application is based on Japanese Patent Application No. 2005-358469 filed on December 13, 2005 and Japanese Patent Application No. 2006-328952 filed on December 6, 2006, and the contents are incorporated herein by reference.

Further, all references cited herein are incorporated in their entireties.

## INDUSTRIAL APPLICABILITY

According to the adhesive pharmaceutical preparation of the invention, the compatibility of a PIB pressure-sensitive adhesive with a drug which is liquid at room temperature or around room temperature can be specifically increased by containing a branched monoalcohol having from 12 to 28 carbon atoms as a solubilizing agent in the pressure-sensitive adhesive layer. As a result, not only it becomes possible to increase blending amount of drug but also bleed of drug from the pressure-sensitive adhesive layer can be suppressed and, what is more, the pressure-sensitive adhesion characteristics sufficient from the practical point of view can be obtained. Accordingly, an adhesive pharmaceutical preparation which can achieve compatibility of the pharmacological action with pressure-sensitive adhesion characteristics at high level can be provided.

## Claims

1. An adhesive pharmaceutical preparation which comprises:

   a backing; and
   a pressure-sensitive adhesive layer laminated on one side of the backing, said pressure-sensitive adhesive layer containing a branched monoalcohol having from 12 to 28 carbon atoms, a drug which is liquid at room temperature or around room temperature (wherein free base of bisoprolol is excluded), and a polyisobutylene pressure-sensitive adhesive.

2. The adhesive pharmaceutical preparation according to claim 1, wherein the drug has a logPow value of from -1.0 to 5.0 and a viscosity at 40°C of from 0.05 to 100,000 mPa·s.

3. The adhesive pharmaceutical preparation according to claim 1 or 2, wherein the branched monoalcohol is a primary alcohol.

4. The adhesive pharmaceutical preparation according to any one of claims 1 to 3, wherein the branched monoalcohol is a 2-alkyl-1-alkanol.

5. The adhesive pharmaceutical preparation according to claim 4, wherein the number of carbons of the alkyl group at the 2-position of the 2-alkyl-1-alkanol is 2 or more.

6. The adhesive pharmaceutical preparation according to any one of claims 1 to 5, wherein the branched monoalcohol is at least one kind selected from 2-hexyl-1-decanol, 2-octyl-1-decanol, 2-hexyl-1-dodecanol, 2-octyl-1-dodecanol and 2-decyl-1-tetradecanol.

*FIG. 1*

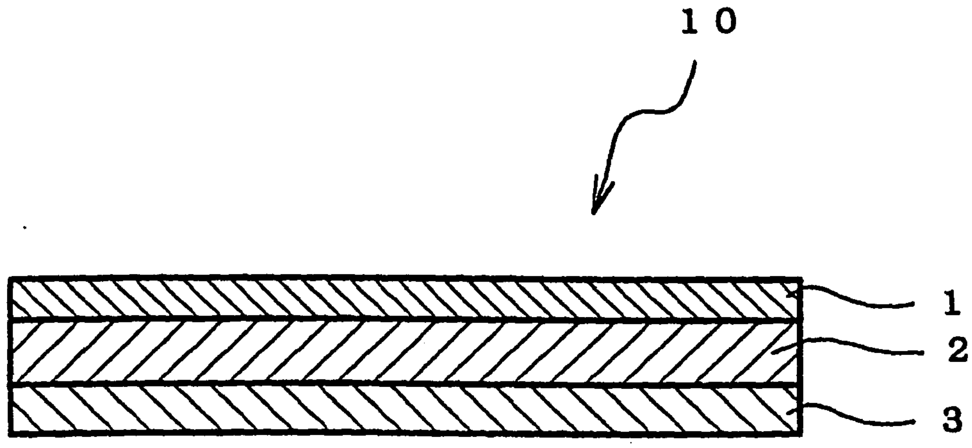

**INTERNATIONAL SEARCH REPORT**

International application No. PCT/JP2006/324875

A. CLASSIFICATION OF SUBJECT MATTER
*A61K9/70*(2006.01)i, *A61K47/10*(2006.01)i, *A61K47/32*(2006.01)i

According to International Patent Classification (IPC) or to both national classification and IPC

B. FIELDS SEARCHED

Minimum documentation searched (classification system followed by classification symbols)
A61K9/70, A61K47/10, A61K47/32

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| P,X | JP 2006-76994 A (Nitto Denko Corp.), 23 March, 2006 (23.03.06), Full text; Claims; Par. No. [0017]; experimental examples 1 to 3 & EP 1625845 A1 & US 2006/0034900 A1 & CA 2514653 A1 | 1-6 |
| P,X | JP 2006-22057 A (Nitto Denko Corp.), 26 January, 2006 (26.01.06), Full text; Claims; Par. Nos. [0012], [0016]; example 6 (Family: none) | 1-6 |

☒ Further documents are listed in the continuation of Box C.　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 March, 2007 (26.03.07) | 10 April, 2007 (10.04.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/324875

C (Continuation). DOCUMENTS CONSIDERED TO BE RELEVANT

| Category* | Citation of document, with indication, where appropriate, of the relevant passages | Relevant to claim No. |
|---|---|---|
| P,X | JP 2006-75588 A (Nitto Denko Corp.), 23 March, 2006 (23.03.06), Full text; Claims; Par. Nos. [0023], [0027]; examples 2, 3 & EP 1625846 A2 & US 2006/0034901 A1 & CA 2514636 A1 | 1-6 |
| X | JP 2004-502725 A (Hexal AG.), 29 January, 2004 (29.01.04), Full text; Claims; Par. Nos. [0016] to [0028], [0029]; examples 1 to 3 & WO 02/03969 A2 & DE 10033853 A1 & CA 2415658 A1 & EP 1301179 A1 & US 2004/086552 A1 | 1-6 |
| A | JP 2004-307364 A (Yutoku Pharmaceutical Ind. Co., Ltd.), 04 November, 2004 (04.11.04), Full text; Claims; Par. Nos. [0017], [0020]; examples 5, 8 (Family: none) | 1-6 |
| A | JP 2002-542277 A (LTS Lohman Therapie-Systeme AG.), 10 December, 2002 (10.12.02), Full text; Claims 10, 24; Par. No. [0055] & DE 19918106 A1 & CA 2370019 A1 & WO 00/64418 A1 & EP 1171104 A2 | 1-6 |
| A | JP 2003-300873 A (Nitto Denko Corp.), 21 October, 2003 (21.10.03), Full text; Claims; Par. Nos. [0010], [0019], [0021], [0023]; examples 3, 8 & WO 03/86376 A1 & AU 2003/227485 A1 & EP 1498118 A1 & US 2005/0214352 A1 | 1-6 |
| A | JP 2003-300868 A (Nitto Denko Corp.), 21 October, 2003 (21.10.03), Full text; Claims; Par. Nos. [0011], [0020], [0022], [0024]; examples 3, 9 & EP 1352649 A1 & CA 2425231 A1 & US 2003/0203697 A1 | 1-6 |
| P,A | JP 2006-206454 A (Nitto Denko Corp.), 10 August, 2006 (10.08.06), Full text; Claims; Par. Nos. [0010], [0020]; examples 1 to 15 (Family: none) | 1-6 |
| A | JP 7-33665 A (Kanebo, Ltd.), 03 February, 1995 (03.02.95), Full text; Claims; Par. No. [0025]; examples 6, 7 (Family: none) | 1-6 |

Form PCT/ISA/210 (continuation of second sheet) (April 2005)

**INTERNATIONAL SEARCH REPORT**

International application No.
PCT/JP2006/324875

C (Continuation). DOCUMENTS CONSIDERED TO BE RELEVANT

| Category* | Citation of document, with indication, where appropriate, of the relevant passages | Relevant to claim No. |
|---|---|---|
| A | JP 4-321624 A (HISAMITSU PHARM CO., INC.), 11 November, 1992 (11.11.92), Full text; Claims; examples 9 to 21 & JP 2004-43512 A | 1-6 |
| A | WO 05/102306 A1 (HISAMITSU PHARM CO., INC.), 03 November, 2005 (03.11.05), Full text; Claims; examples 2 to 6 (Family: none) | 1-6 |
| A | WO 02/98396 A1 (HISAMITSU PHARM CO., INC.), 12 December, 2002 (12.12.02), Full text; Claims; examples 24, 30 & CA 2448689 A1 & EP 1400240 A1 & US 2004/146548 A1 | 1-6 |
| A | WO 05/72716 A1 (HISAMITSU PHARM CO., LTD.), 11 August, 2005 (11.08.05), Full text; Claims 1, 7; Par. No. [0030] (Family: none) | 1-6 |

Form PCT/ISA/210 (continuation of second sheet) (April 2005)

**INTERNATIONAL SEARCH REPORT**

International application No.
PCT/JP2006/324875

<Regarding the scope of search>

What is illustrated by showing a specific chemical structure in the specification and is actually confirmed in terms of bleed resistance, anchor effect, the balance and/or favorability of stickiness and adhesiveness of adhesive preparation adopted and prepared specifically in Examples as the "branched monohydric alcohol having 12 to 28 carbon atoms" in claim 1 is only a part of primary alcohols corresponding to 2-alkyl-1-alkanol among the branched monohydric alcohols satisfying the definition according to the above carbon number ([0018]-[0020], Examples 1-6). Further, when taking into account the point that it can be understood whether the covering effect in which a difference in the desired effect can be caused depending on the branching degree of carbon chains and the exposure degree of hydroxyl groups is high based on the difference in the effect related to bleed resistance or adhesiveness of the effects in the case of adopting 2-hexyl-1-decanol which is one example of the 2-alkyl-1-alkanol and the case of adopting hexadecane-8-ol with a similar structure ([0019], [Table 2]), an adhesive preparation other than the case where the above 2-alkyl-1-alkanol is adopted as the monohydric alcohol specified in claims 1-3 cannot be sufficiently supported in the specification within the meaning of PCT Article 6, or cannot be sufficiently disclosed in the specification to such an extent that a person skilled in the art can fully understand and carry out within the meaning of PCT Article 5.

Accordingly, a search was made only for the embodiment of the adhesive preparation obtained by adopting 2-alkyl-1-alkanol specified in any one of claims 4-6 as the "branched monohydric alcohol having 12 to 28 carbon atoms" among the adhesive preparations having an adhesive layer containing a polyisobutylene adhesive specified in claims 1-3.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005023088 A
- JP 2005358469 A
- JP 2006328952 A